(19) Europäisches Patentamt
European Patent Office
Office européen des brevets

(11) Publication number: 0 260 901 B1

(12) # EUROPEAN PATENT SPECIFICATION

(45) Date of publication of patent specification: 06.03.91 (51) Int. Cl.5: C07C 311/44, A61K 31/18

(21) Application number: 87308082.4

(22) Date of filing: 14.09.87

The file contains technical information submitted after the application was filed and not included in this specification

(54) **Benzenesulphonamides useful as anti-arrythmic agents.**

(30) Priority: 18.09.86 US 909133
02.05.87 GB 8710529

(43) Date of publication of application:
23.03.88 Bulletin 88/12

(45) Publication of the grant of the patent:
06.03.91 Bulletin 91/10

(84) Designated Contracting States:
AT BE CH DE ES FR GR IT LI LU NL SE

(56) References cited:
EP-A- 0 158 775
EP-A- 0 167 245
DE-A- 2 623 447
US-A- 4 587 360

(73) Proprietor: AMERICAN HOME PRODUCTS CORPORATION
685, Third Avenue
New York, New York 10017(US)

(72) Inventor: Buzby, George Carroll, Jr.
997 Stoneybrook Drive Blue Bell
Montgomery County Pennsylvania(US)
Inventor: Colatsky, Thomas John
68 Birch Lane Paoli
Chester County,Pennsylvania(US)

(74) Representative: Connelly, Michael John et al
C/o Wyeth Laboratories Huntercombe Lane
South Taplow
Maidenhead Berkshire, SL6 0PH(GB)

EP 0 260 901 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

### Background of the Invention

Class III anti-arrhythmic agents may be categorized as having the ability to markedly prolong dog Purkinje fiber action potential duration without producing significant changes in maximal upstroke velocity. Unlike Class I anti-arrhythmic agents, a pure Class III agent displays no effects on cardiac sodium channels. The electrophysiologic properties of a compound defining a Class III activity profile are observed in vivo as negligible effects on atrial, ventricular and H-V conduction lines while producing a marked increase (greater than 20 percent) in both the atrial and ventricular refractory period. In contrast, Class I agents will demonstrate a marked slowing of ventricular conduction velocity, generally without significant changes in the refractory period. Recent reviews of these agents are by Bexton et al., Pharmac. Ther. 17 , 315-55 (1982); VaughanWilliams, J. Clin. Pharmacol. 24 , 129-47 (1984) and Thomis et al., Ann. Rep. Med. Chem. 18 , 99-108 (1983).

German Offenlegungsschrift 1912848 discloses in Example 5 the intermediate $N^1$-(2-isopropylaminoethyl)-$N^4$-acetyl-sulfanilamide which is used to produce 1-sulfanilyl-2-imino-3-isopropyl-imidazolidin said to be useful as a hypoglycemic agent.

Silberg et al., ACAD Rep. Populace Romire, Fillala Clug, Studee Cercetari Med., 10 244-52 (1959) discloses p-acetylamino-N-(2-diethylamino-ethyl) benzenesulfonamide among other compounds compared in their anti-arrhythmic properties with procainamide.

The Abstracts of Papers to be presented at the 192nd ACS National Meeting, September 7-12, 1986 at Anaheim, California reports Abstract 9 by R. A. Wohl et al. which discloses N-[2-(diethylamino)ethyl]-4-[-(methylsulfonyl)-amino] benzamide hydrochloride projected as a potential Class III anti-arrhythmic agent.

EPO 158775A discloses a class of substituted sulphonamidobenzamides including N-[2-(diethylamino)-ethyl]-4-[methylsulphonyl)amino] benzamide hydrochloride and their use as antiarrhythmic agents. The corresponding class of substituted sulphonamidobenzenesulphonamides are said to be contemplated as equivalents. In particular N-[2-(diethylamino)ethyl]-4-[(methylsulphonyl)amino] benzenesulphonamide is said to be construed as a Class III antiarrhythmic agent.

### Description of the Invention

This invention provides new compounds classified by their pharmacological profile as Class III anti-arrhythmic agents. The compounds are those having the formula

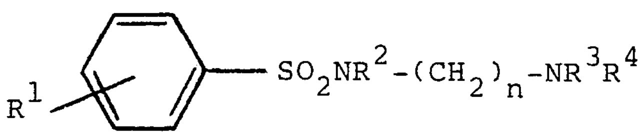

(I)

in which $R^1$ is alkylsulphonamido of 1 to 6 carbon atoms; $R^2$ is alkyl of up to 6 carbon atoms but not methyl; $R^3$ is hydrogen or alkyl of 1 to 6 carbon atoms; $R^4$ is alkyl of 1 to 6 carbon atoms; and n is one of the integers 2,3 or 4; or a pharmaceutically acceptable salt thereof.

The compounds where $R^1$ is $CH_3SO_2NH$- are preferred. The most preferred compounds are embraced by the structual formula:

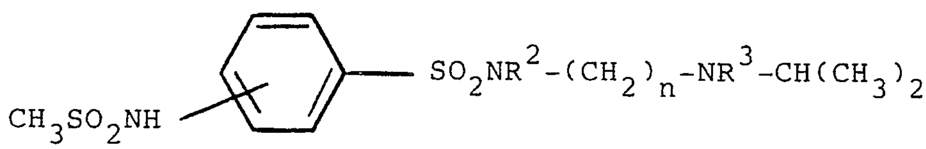

(II)

where $R^2$ is straight or branched alkyl of up to 6 carbon atoms but not methyl; $R^3$ is hydrogen or isopropyl; and n is one of the integers 2,3 or 4; or a pharmaceutically acceptable salt thereof.

The pharmaceutically acceptable salts of the compounds of this invention are prepared directly by neutralization of the free base. These physiologically acceptable salts may be formed with organic or inorganic acids such as hydrochloric, hydrobromic, phosphoric, sulphuric, sulphamic, nitric, methylsulphonic, acetic, maleic, succinic, fumaric, tartaric, citric, salicylic, lactic, naphthalenesulphonic acid and the like.

The compounds of the present invention contain two sulphonamide functions and a secondary or tertiary amine function. They may therefore be prepared by various methods available for the preparation of sulphonamides (sulphonylation of amines) and for the preparation of secondary and tertiary amines (alkylation of amines, reductive amination of carbonyl compounds, reduction of amides or Schiff's bases).

In the following description relating to a process for the preparation of the new compounds of the invention the symbols $R^1$, $R^2$, $R^3$, $R^4$ and n are as defined above. The present invention provides a process for the preparation of a compound having formula I or a pharmaceutically acceptable salt thereof, in which

(a) an amine having the formula III

$$H\overset{*}{N}R^2-(CH_2)_n-NR^4R^5 \quad (III)$$

(where $R^5$ is $R^3$ or a removable protecting group) is sulphonylated at the nitrogen atom marked with an asterisk by reaction with a sulphonylating agent for introducing a sulphonyl group having the formula IV

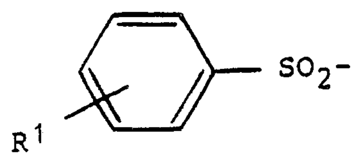

(IV)

and, where necessary, a protecting group $R^5$ is removed; or

(b) an amine having the formula V

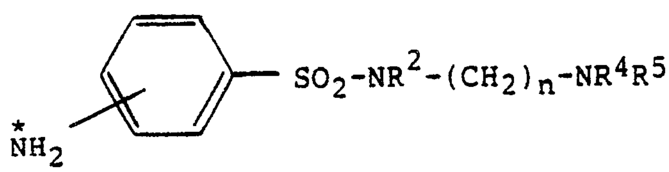

(V)

(where $R^5$ is as defined above) is sulphonylated at the nitrogen atom marked with an asterisk by reaction with a sulphonylating agent for introducing a alkylsulphonyl group of 1 to 6 carbon atoms, an arylsulphonyl group of 6 to 10 carbon atoms or a perfluoroalkylsulphonyl group of 1 to 6 carbon atoms and, where necessary, a protecting group $R^5$ is removed; or

(c) an amine having the formula VI

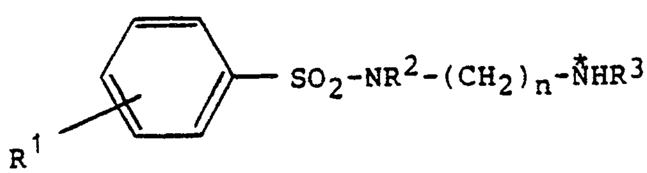

(VI)

is alkylated at the nitrogen atom marked with an asterisk by reaction with an alkylating agent for introducing a $C_4$-$C_6$ alkyl group; or

(d) an amine having the formula $HNR^3R^4$ is alkylated with an alkylating agent for introducing a substituted alkyl group having the formula VII

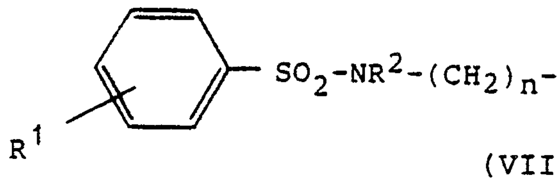

;or

(VII)

(e) a carbonyl compound having the formula $C_mH_{2m}+1$-CO-$C_pH_{2p}+1$ (where m and p are independently 0, 1, 2, 3, 4 or 5 and the total of m and p is 0, 1, 2, 3, 4 or 5) is subjected to reductive amination by reaction with an amine having the formula VI as shown above; or

(f) an aldehyde having the formula VIII

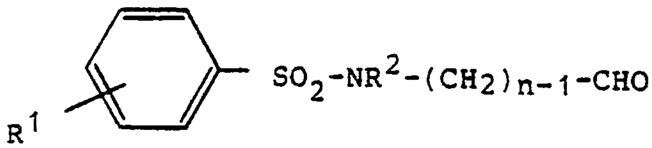

(VIII)

is subjected to reductive amination by reaction with an amine having the formula $HNR^3R^4$; or

(g) a Schiff's base having the formula IX or X

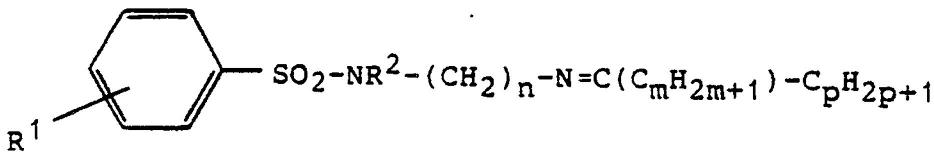

(IX)

(where m and p are as defined above)

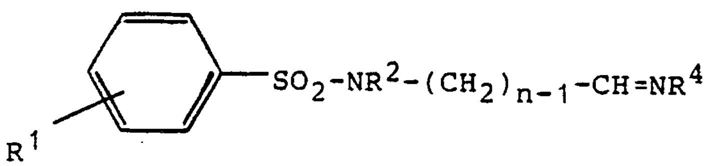

(X)

or an amide having the formula XI or XII

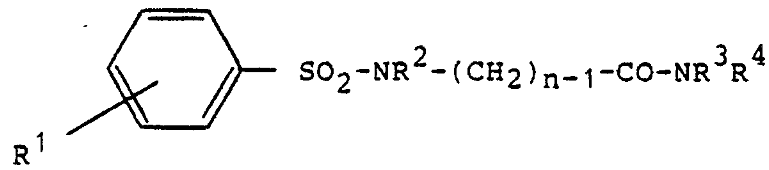

(XI)

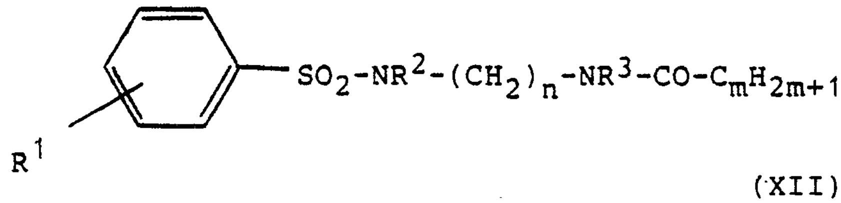

(XII)

(where m is as defined above) is reduced to form an amine. The process of the invention may also include addition of an acid to a free base form of compound of formula I to form an acid addition salt or neutralisation of an acid addition salt by subtraction of an acid to form a free base form of the compound of formula I.

The reactants used in steps (a) to (g) are known compounds or, if new, can be prepared by methods known per se .

Sulphonylation step (a) may be carried out in manner known for the sulphonylation of amines. As sulphonylating agent one may use the appropriately substituted benzenesulphonyl chloride, particularly the sulphonyl chloride, but the sulphonic acid anhydride may be used instead. The sulphonylation is preferably carried out in the presence of an acid binding agent such as a tertiary amine, for example triethylamine. Where $R^5 = R^3 = C_1$-$C_6$ alkyl, then only one nitrogen atom is available for sulphonylation and a single sulphonylation product is obtained. However, where $R^5 = R^3 = H$ the amine of formula III has two nitrogen atoms that may react with the sulphonylating agent and one may find that monosulphonylation yields a mixture of two sulphonamides. One can avoid such a mixture by using an amine having formula III in which $R^5$ represents a removable protecting group. The protecting group is removed after the sulphonylation to yield an end compound where $R^3$ is hydrogen. As examples of protecting groups there may be mentioned alkoxycarbonyl groups (which can be removed by acid or base hydrolysis), fo r example t -butoxycarbonyl (which may be removed by hydrogenation) or phthalimido (which may be removed by reaction with methylamine).

Step (b) may also be carried out in manner known for the sulphonylation of amines. The sulphonylating agent may be a sulphonyl halide, particularly the sulphonyl chloride or it may be the sulphonic acid anhydride such as trifluormethanesulphonic anhydride or the like. The sulphonylation is preferably carried out in the presence of an acid binding agent, for example, triethylamine. Step (b) may be carried out as part of a three stage synthesis. In the first stage an amine having formulae III is sulphonylated with a sulphonylating agent for introducing a sulphonyl group having the formula XIII

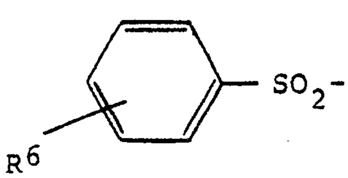

(XIII)

where $R^6$ represents a precursor for $-NH_2$. In the second stage the precursor for $-NH_2$ is converted into $-NH_2$ so as to form the amine of formula V. The third stage is step (b).

As precursor for $-NH_2$ there may be used a nitro group which can be reduced to the amino group by catalytic hydrogenation. Alternatively the precursor may be $-NHR^7$ where $R^7$ is a protecting group, for example, one of the protecting groups mentioned under $R^5$.

Where an end compound in which $R^3$ is hydrogen is to be prepared by such a synthesis, it is

recommended to use a removable protecting group as $R^5$ in the amine starting material of formula III and to retain the protecting group until after the second of the two sulphonylation reactions. This ensures that only one nitrogen atom is sulphonylatable during each of the sulphonylation reactions and avoids the possibility of undesired sulphonylation at the nitrogen atom bearing $R^4$. When $R^5$ is a protecting group in the amine starting material of formula III, then $R^6$ should be so chosen that the reaction conditions for the conversion of $R^6$ into $-NH_2$ do not simultaneously remove the protecting group from the nitrogen atom bearing $R^4$ so that this nitrogen atom is protected during step (b).

The amines having the formula VI to be used as reactants in steps (c) and (e) are primary amines (where $R^3$ is hydrogen) and secondary amines (where $R^3$ is alkyl of 1 to 6 carbon atoms). The secondary amines are, of course, identical to the secondary amines of formula I where $R^3$ is hydrogen and $R^4$ is alkyl of 1 to 6, carbon atoms and can therefore be prepared as described above with reference to steps (a) and (b). The primary amines can be prepared by reacting an amine having the formula XIV

$$HNR_2-(CH_2)_n-NHR^6 \qquad (XIV)$$

(where $R^6$ is a removable protecting group) with the same sulphonylating agent as used in step (a) and removing the protecting group in a subsequent step.

The alkylating agent used in step (d) is preferably a compound having the formula Y-Z where Y is the group having formula VII and Z is a leaving group, for example, tosyloxy, chloro or bromo. The alkylating agent may be prepared by adding a compound having the formule XV

$$HNR^2-(CH_2)_n-Z \qquad (XV)$$

in the form of a hydrohalide to a solution of the sulphonylating agent used in step (a) with an acid binding agent.

The aldehyde having the formula VIII as used in step (f) may be prepared by sulphonylating an amine having the formula XVI

$$HNR^2-(CH_2)_{n-1}-R^7 \qquad (XVI)$$

[where $R^7$ is a protected form of a -CHO group, for example, a group of the formula

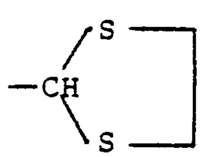

(XVII)

or an acetal derivative such as $-CH(OCH_3)_2$] with the same sulphonylating agent as used in step (a) with subsequent removal of the protection. The group of formula XVII can be converted into -CHO by means of various reagents including, for example, $Hg(CF_3CO_2)_2$. The group $-CH(OCH_3)_2$ can be converted into -CHO under acid conditions. The Schiff's base of formula X to be used in step (g) can be prepared by reaction of the aldehyde of formula VIII with a amine of formula $H_2NR^4$. The Schiff's base of formula IX can be prepared by reaction of an amine of formula VI where $R^3$ is hydrogen with the carbonyl compound identified under step (e). The amides of formulae XI and XII as used in step (g) may be prepared by sulphonylation of an amine having the formula XIa or XIIa with the sulphonylating agent of step (a).

$$HNR^2-(CH_2)_{n-1}-CO-NR^3R^4 \quad (XIa)$$

$$HNR^2-(CH_2)_n-NR^3-CO-C_mH_{2m+1} \quad (XIIIa)$$

Alkylation steps (c) and (d) may be carried out in known manner for alkylation of amines. The amine may be reacted with the alkylating agent in the presence of a suitable base, for example, triethylamine or an excess of the amine reactant. The reaction conditions should not be too strongly basic since otherwise alkylation may take place at a sulphonamide nitrogen atom.

Reductive amination of carbonyl compounds according to steps (e) and (f) may be carried out with catalytic hydrogen, nascent hydrogen or the Leuckart reaction. Reduction of amides according to step (g) may be carried out with borane as reducing agent. Reduction of Schiff's bases according to step (g) may be carried out by catalytic hydrogenation.

The compounds of formula I and their pharmaceutically acceptable salts are useful as pharmaceuticals and, in particular, are anti-arrhythmic agents. The invention includes a pharmaceutical composition comprising such a compound in combination or association with a pharmaceutically suitable carrier. The composition may be prepared by bringing the compound into association or combination with the carrier.

The compounds of this invention demonstrate anti-arrhythmic activity when tested in the standard experimental animal in accordance with the following procedure:

Miniature pigs of either sex weighing 11-24 kg were anesthetized by administration of 35 mg/kg sodium pentobarbital i.p. and ventilated with room air following tracheotomy using a Harvard respirator pump set to deliver 20 ml/kg at a rate of 20/min. The left femoral artery and vein were cannulated for the recording of blood pressure and for drug administration, respectively. Blood pressure and lead II EKG were recorded on a Beckman RM dynograph recorder (Model R-612).

The heart was exposed by a left thoracotomy performed at the fifth intercostal space. A silk ligature was placed beneath the left anterior descending coronary artery (LAD) about 1 cm from its origin and distal to the septal artery branch. The artery was occluded by lifting the vessel with the ligature and quickly placing a bull-dog clamp (3 X 12 mm pudded jaws) over the artery. The clamp remained in place for a period of 20 min. Removal of the clamp produced a rapid reperfusion of the ischemic myocardium as evidenced by the return of normal color to the myocardium distal to the site of occlusion. Ectopic activity was monitored during occlusion and reperfusion by recording the lead II EKG at chart speeds of 5-25 mm/s. Animals were allowed to stabilize for at least 30 min prior to drug administration.

Pigs were randomized into groups receiving either vehicle or test drug at 5 mg/kg i.v. Animals surviving the period of occlusion were subsequently reperfused. No attempt was made to resuscitate animals experiencing ventricular fibrillation (VF) at any time following occlusion. Efficacy was established by noting the rate of survival of treatment vs. control groups using Fisher's exact test or Mantel-Haenszel test for the survival curves. In the absence of treatment, less than 30 percent of the animals survive the period of occlusion, with a mean time to death onset of 8-12 min. An effective compound either prevented death or prolonged survival time.

The compounds of this invention display a Class III anti-arrhythmic profile. Of these, the products of Examples 1 and 4 are representative. The Class III antia rrhythmic activity was established in accordance with the following standard test procedure:

Bundles of free-running Purkinje fibers with attached myocardium obtained from either ventricle of adult dog heart were pinned without stretching to the bottom of a 10 ml tissue chamber and continuously superfused with oxygenated Tyrode's solution at a flow rate of 10 ml/min. The composition of the Tyrode's solution was (mM): NaCl 150; KCl 4.0; $CaCl_2$ 2.7; $MgCl_2$ 0.5; HEPE buffer (7.4) 10; dextrose 5.5. The solution was aerated with 100% $O_2$. Bath temperature was maintained at 36 ± 0.5° C by circulating the superfusate through a thermostatically controlled water bath immediately prior to entering the tissue chamber.

The preparations were stimulated through bipolar Teflon-coated platinum wires, bared at the tips, placed on the endocardial surface of the attached myocardium, using a W.P.I. digital stimulator set to deliver constant current pulses 1-2 msec in duration at cycle lengths (c.l.) of 330 or 1000 msec. Stimulus strength was set at approximately 2x diastolic threshold, and adjusted as required throughout the experiment. All preparations were allowed to equilibrate in the tissue chamber for at least 1 hour before measurements were begun. Subsequently, a minimum of 60 minutes was allowed for equilibration with each drug-containing superfusate before post-drug measurements were made. Impalements were made at 6-10

sites throughout the preparation before and after drug exposure. Offset potentials were re-checked at the conclusion of each experiment.

Glass microelectrodes filled with 3M KCl were coupled to high impedance negative capacitance electrometers (W.P. Instruments, New Haven, CT), and Ag/AgCl half-cells used as reference electrodes. The first derivative of the action potential upstroke (V max) was obtained using an analog differentiator circuit, coupled to a peak-hold circuit that retained the recorded value of Vmax for 30-70 msec. Action potential and Vmax tracings were displayed on a Tektronix storage oscilloscope, and photographed for later analysis. In addition, chart paper recordings of Vmax were obtained using the peak-hold device output.

Fresh stock solutions of drug were prepared for each experiment. Compounds were dissolved in distilled water at total concentrations of 1-10 mg/ml, and subsequently diluted to a final concentration of 3 μM in appropriate volumes of normal Tyrode's solution for evaluation.

Action potential (AP) parameters measured included: diastolic take-off potential (or activation voltage, $V_{act}$); AP overshoot ($V_{os}$); AP duration measured as the time taken to repolarize to -20 mV ($APD_{20}$), -60 mV ($APD_{60}$), and -80 mV and ($APD_{80}$); and maximal upstroke velocity (Vmax). Data were compared using a two-sample t-test, with statistical significance taken as $p < 0.05$. An increase in $APD_{60}$ that occurred without a significant change in Vmax was taken, by definition, to indicate Class III anti-arrhythmic activity.

Based upon the activity profile elicited by the compounds of this invention in the above-described standard scientifically recognized test models, the compounds are established as anti-arrhythmic agents useful in the treatment of cardiac arrhythmias and conditions characterized by coronary arteries vasospasm. For that purpose, the compounds may be administered orally or parenterally in suitable dosage forms compatable with the route of administration, whether oral, intraperitoneal, intramuscular, intravenous, intranasal, buccal, etc. The effective dose range determined in the animal test models has been established at from about 1 to about 5 milligrams per kilogram host body weight (preferably from 2 to 10 mg/kg), i.v., and from about 2 to about 10 mg/kg (preferably 5 to 20 mg/kg) p.o., to be administered in single or plural doses as needed to relieve the arrhythmatic dysfunction. The specific dosage regimen for a given patient will d nd upon age, pathological state, severity of dysfunction, size of the patient, etc. Oral administration is performed with either a liquid or solid dosage unit in any conventional form such as tablets, capsules, solutions, etc., which comprise a unit dose (e.g. from about 50 milligrams to about 400 milligrams) of the active ingredient alone or in combination with adjuvants needed for conventional coating, tableting, solubilizing, flavoring or coloring. Parenteral administration with liquid unit dosage forms may be via sterile solutions or suspensions in aqueous of oleagenous medium. Isotonic aqueous vehicle for injection is preferred with or without stabilizers, preservatives and emulsifiers.

The following two examples illustrate the preparation of compounds of this invention. After example 1, the change in action potential duration and upstroke velocity, the time to death and percent survival, which where tested, are provided.

## Example 1

N-(1-Methylethyl)-N-[2-[bis(1-methylethyl)amino]ethyl] -4-(methylsulphonamido)benzensulphonamide

4 -Methylsulphonamidobenzensulphonyl chloride (16.45g, .061 moles) was added portionwise as a solid over one hour to a solution of N,N,NI-tri-isopropylethylenadiamine (11.36g, .061 moles) and triethylamine (6.16g, .061 moles) in methylene chloride (300ml). After addition, the reaction was stirred one hour, washed with $NaHCO_3$ solution then brine and the solvent was removed. The gummy residue was chromatographed on dry column silica gel (400g) using 10% MeOH/EtOAc to give the pure product free base (10.1g). Treatment of the free base with isopropanolic HC1 gave the monohydrochloride salt as a white solid, (11.92g)-m.r 209-211 °C.

Analysis for: $C_{18}H_{34}ClO_4N_3S_2$

Calculated: C,47.41;H,7.51;N,9.21

Found: C,47.16; H,7.29; N,8.83

3μM, 1000 msec c.l.: %$\Delta APD_{60}$=35; % ΔVmax=8

Time to death: 18.3 min; 67% survival

Example 2

N-(1-methylethyl)-N-[2-[(1-methylethyl)-amino]ethyl] -4-(methylsulphonamido)benzenesulphonamide Hydrochloride

4-Methylsulphonamidobenzenesulphonyl chloride (2.70 g, 0.01m) was added portionwise as a solid over 1 hour to a stirred solution of N,N[1]-diisopropylethylene diamine (2.88g, 0.02m) in 75ml of methylene chloride. The white preceipitate that formed was washed with methylene chloride and then with water to give 1.94 g of a white solid (mp. 190-195 °C). The solid was suspended in about 50 ml of boiling isopropyl alcohol and isopropyl alcohol saturated with HC1 was added until all the solid dissolved. On cooling crystals formed, and after filtering, washing and drying, 1.47 g of the title compounds (mp. 220-222 °C) was obtained.

## Claims

1. A compound having the formula I

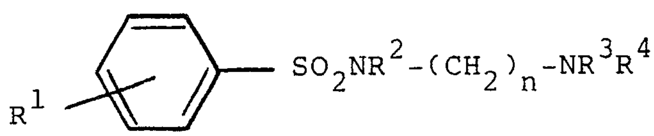

(I)

wherein $R^1$ is alkylsulphonamido of 1 to 6 carbon atoms; $R^2$ is alkyl of up to 6 carbon atoms but not methyl; $R^3$ is hydrogen or alkyl of 1 to 6 carbon atoms; $R^4$ is alkyl of 1 to 6 carbon atoms; and n is one of the integers 2,3 and 4; or a pharmaceutically acceptable salt thereof.

2. A compound as claimed in Claim 1, in which $R^1$ is $CH_3SO_2NH$-.

3. A compound as claimed in Claim 1 or Claim 2, wherein $R^3$ is alkyl of 1 to 6 carbon atoms.

4. A compound having the formula II

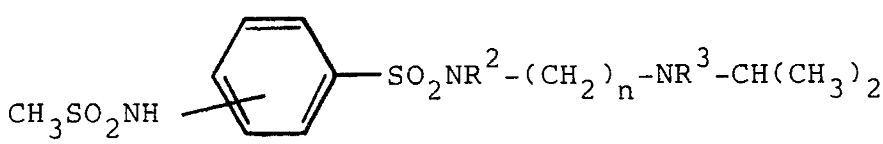

II

where $R^2$ is straight or branched chain alkyl of up to 6 carbon atoms but not methyl; $R^3$ is isopropyl; n is one of the integers 2,3 and 4; or a pharmaceutically acceptable salt thereof.

5. N(1-methylethyl)-N-[2-[bis(methylethyl) amino]ethyl]-4-(methylsulphonamido)benzenesulphonamide or a pharmaceutically acceptable salt thereof.

6. A compound as claimed in Claim 1 or Claim 2, wherein $R^3$ is hydrogen.

7. A compound having formula II

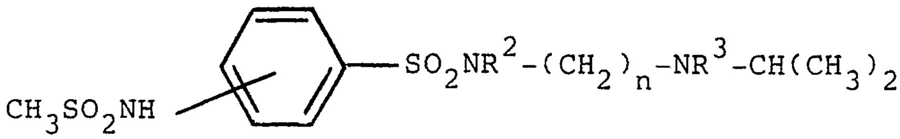

(II)

wherein $R^2$ is straight or branched chain alkyl of up to 6 carbon atoms but not methyl; $R^3$ is hydrogen; n is one of the integers 2,3 and 4; or a pharmaceutically acceptable salt thereof.

**8.** N-(1-Methylethyl)-N-[2-[(1-methylethyl)amino] ethyl]-4-(methylsulphonamido)benzenesulphonamide or a pharmaceutically acceptable salt thereof.

**9.** A process for the preparation of a compound having the fomula I

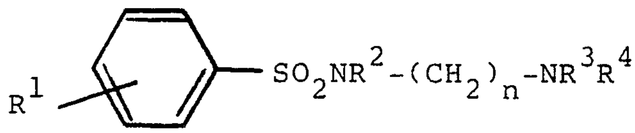

(I)

(wherein $R^1$, $R^2$, $R^3$, $R^4$ and n are as defined in Claim 1) or a pharmaceutically acceptable salt thereof, in which

a) an amine having the formula III

$$H\overset{*}{N}R^2-(CH_2)_n-NR^4R^5$$

(III)

(where $R^2$, $R^4$ and n are as defined above and $R^5$ is $R^3$ or a removable protecting group) is sulphonylated at the nitrogen atom marked with an asterisk by reaction with a sulphonylating agent for introducing a sulphonyl group having the formula IV

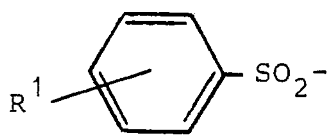

(IV)

(where $R^1$ is defined above)
and where necessary, a protecting group $R^5$ is removed; or
(b) an amine having the formula V

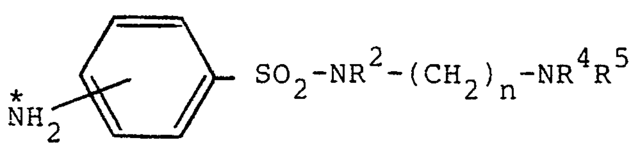

(V)

(where $R^2$, $R^4$, $R^5$ and n are as defined above)
is sulphonylated at the nitrogen atom marked with an asterisk by reaction with a sulphonylating agent for introducing a alkylsulphonyl group of 1 to 6 carbon atoms, an arylsulphonyl group of 6 to 10 carbon atoms or a perfluoroalkylsulphonyl group of 1 to 6 carbon atoms and, where necessary, a protecting group $R^5$ is removed; or
(c) an amine having the formula VI

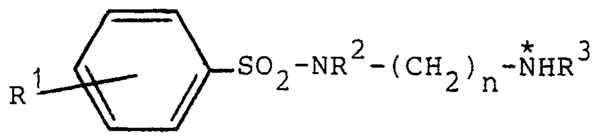

(VI)

(where $R^1$, $R^2$, n and $R^3$ are as defined above) is alkylated at the nitrogen atom marked with an asterisk by reaction with an alkylating agent for introducing a $C_1$-$C_6$ alkyl group; or
(d) an amine having the formula $NHR^3R^4$ (where $R^3$ and $R^4$ are as defined above) is alkylated with an alkylating agent for introducing a substituted alkyl group having the formula VII

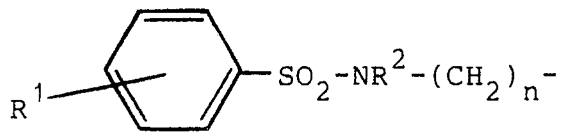

(VII)

(where $R^1$, $R^2$ and n are as defined above); or
(e) a carbonyl compound having the formula $C_mH_{2m+1}$-CO-$C_pH_{2p+1}$ (where m and p are independently 0, 1, 2, 3, 4 or 5 and the total of m and p is 0,1,2, 3, 4 or 5) is subjected to reductive amination by reaction with an amine having the formula VI as shown and defined above; or
(f) an aldehyde having the formula VII

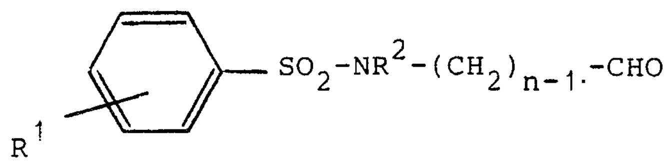

(VIII)

(where $R^1$, $R^2$ and n are as defined above) is subjected to reductive amination by reaction with an amine having the formula $HNR^3R^4$ (where $R^3$ and $R^4$ are as defined above); or
(g) a Schiff's base having the formula IX or X

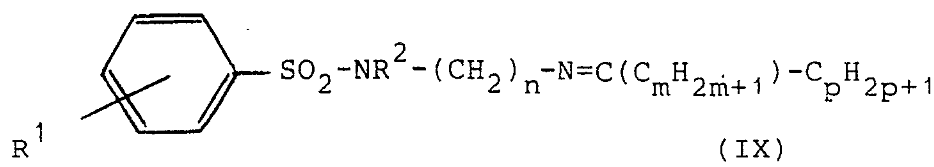

(IX)

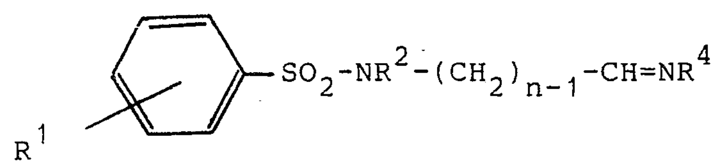

(X)

(where $R^1$, $R^2$, $R^4$, n, m and p are as defined above)
or an amide having the formula XI or XII

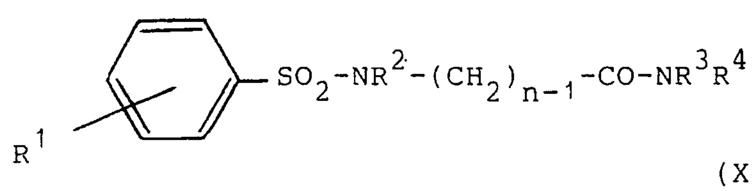

(XI)

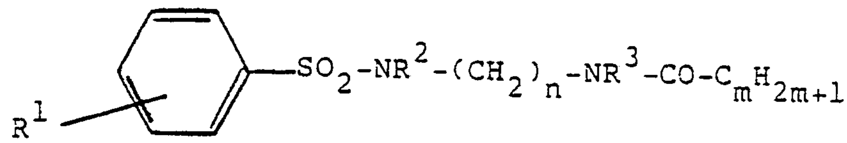

(XIII)

(where $R^1$, $R^2$, $R^3$, $R^4$, n and m are as defined above) is reduced to form an amine; and if deisred an acid may be added to a free base form of compound of formula I to form an acid addition salt or an acid addition salt may be neutralized by subtraction of an acid to form a free base form of the compound of formula I.

Claims for the following Contracting States : AT, ES, GR

1. A process for the preparation of a compound having the formula I

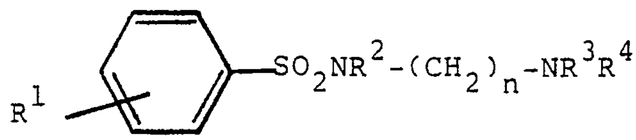

(I)

or a pharmaceutically acceptable salt thereof wherein R1 is alkylsuphonamido of 1 to 6 carbon atoms; $R^2$ is alkyl or up to 6 carbon atoms but not methyl; $R^3$ is hydrogen or alkyl of 1 to 6 carbon atoms; $R^4$ alkyl of 1 to 6 carbon atoms and n is one of the integers 2,3 and 3; in which;

(a) an amine having the formula III

$$H\overset{*}{N}R^2-(CH_2)_n-NR^4R^5$$

(where $R^2$, $R^4$ and n are as defined above and $R^5$ is $R^3$ or a removable protecting group 7) is sulphonylated at the nitrogen atom marked with an asterisk with a sulphonylating agent for introducing a sulphonyl group having the formula IV

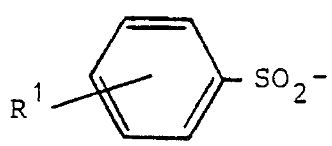

(IV)

(where $R^1$ is defined above) and, where necessary, a protecting group $R^5$ is removed;
or
(b) an amine having the formula V

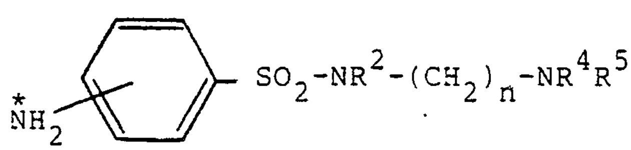

(V)

(where $R^2$, $R^4$, $R^5$ and n are as defined above)
is sulphonylated at the nitrogen atom marked with an asterisk by reaction with a sulphonylating agent for introducing a alkylsulphonyl group of 1 to 6 carbon atoms, an arylsulphonyl group of 6 to 10 carbon atoms or a perfluoroalkylsulphonyl group of 1 to 6 carbon atoms and, where necessary, a protecting group $R^5$ is removed; or

(c) an amine having the formula VI

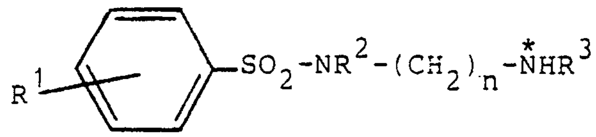

(VI)

(where $R^1$, $R^2$, n and $R^3$ are as defined above) is alkylated at the nitrogen atom marked with an asterisk by reaction with an alkylating agent for introducing a $C_1$-$C_6$ alkyl group; or

(d) an amine having the formula $NHR^3R^4$ (where $R^3$ and $R^4$ are as defined above) is alkylated with an alkylating agent for introducing a substituted alkyl group having the formula VII

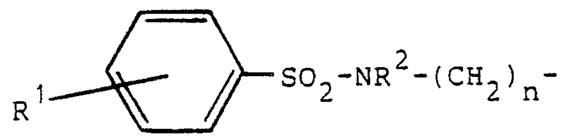

(VII)

(where $R^1$ , $R^2$ and n are as defined above); or

(e) a carbonyl compound having the formula $C_mH_{2m+1}$- CO-$C_pH_{2p+1}$ (where m and p are independently 0, 1, 2, 3, 4 or 5 and the total of m and p is 0,1,2, 3, 4 or 5) is subjected to reductive amination by reaction with an amine having the formula VI as shown and defined above; or

(f) an aldehyde having the formula VIII

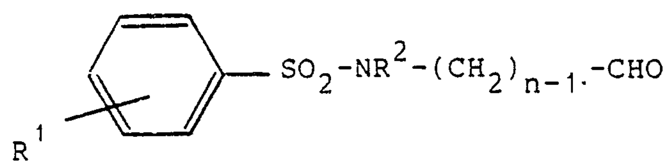

(VIII)

(where $R^1$, $R^2$ and n are as defined above) is subjected to reductive amination by reaction with an amine having the formula $HNR^3R^4$ (where $R^3$ and $R^4$ are as defined above); or

(g) a Schiff's base having the formula IX or X

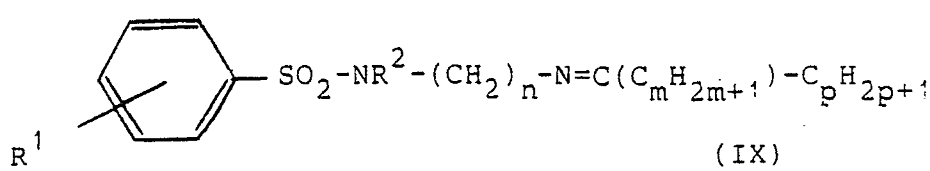

(IX)

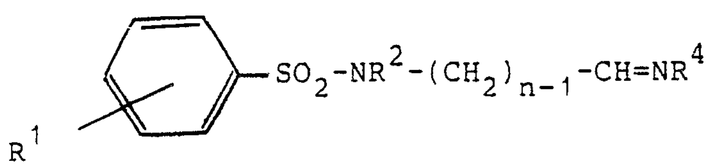

(X)

(where $R^1$, $R^2$, $R^4$, n, m and p are as defined above) or an amide having the formula XI or XII

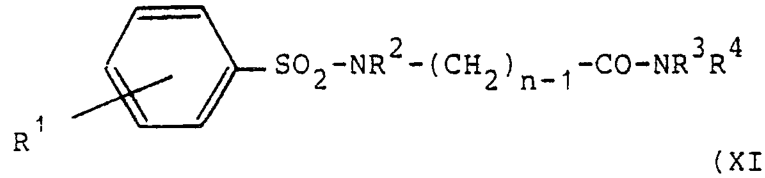

(XI)

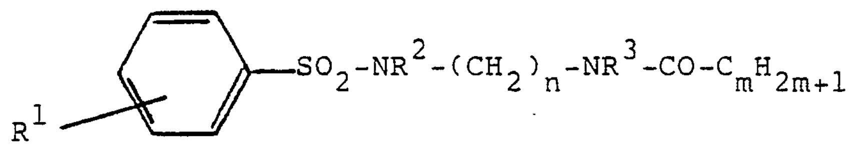

(XIII)

(where $R^1$, $R^2$, $R^3$, $R^4$, n and m are as defined above) is reduced to form an amine; and if desired an acid may be added to a free base form of compound of formula I to form an acid addition salt or an acid addition salt may be neutralized by subtraction of an acid to form a free base form of the compound of formula I.

**2.** A process as claimed in Claim 1, wherein
$R^3$ is hydrogen.

**3.** A process as claimed in Claim 1 or 2, wherein
$R^3$ is alkyl of 1 to 6 carbon atoms.

**4.** A process as claimed in Claim 2 or 3, wherein
$R^1$ is $CH_3SO_2NH$-,

**5.** A process for the preparation of a pharmaceutical composition, wherein a compound having formula I as defined and illustrated in Claim 1 or a pharmaceutically acceptable salt thereof is brought into combination or association with a pharmaceutically acceptable carrier.

**6.** A process as claimed in Claim 5, wherein
$R^3$ is hydrogen.

**7.** A process as claimed in Claim 5, wherein
$R^3$ is alkyl of 1 to 6 carbon atoms.

**8.** A process as claimed in Claim 6 or 7 wherein
$R^1$ is $CH_3SO_2NH$-.

## Revendications

**1.** Composé de formule I :

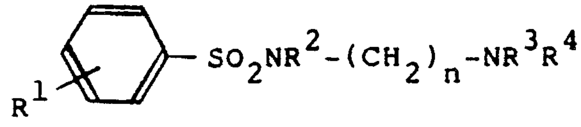

(I)

où $R^1$ est alcoylsulfonamido comptant 1 à 6 atomes de carbone; $R^2$ est alcoyle comptant jusqu'à 6 atomes de carbone, mais non méthyle; $R^3$ est hydrogène ou alcoyle comptant 1 à 6 atomes de carbone; $R^4$ est alcoyle de 1 à 6 atomes de carbone; et n est l'un des entiers 2, 3 et 4; ou un sel pharmaceutiquement acceptable de celui-ci.

2. Composé suivant la revendication 1, dans lequel $R^1$ est $CH_3SO_2NH$-.

3. Composé suivant la revendication 1 ou 2, dans lequel $R^3$ est alcoyle comptant 1 à 6 atomes de carbone.

4. Composé de formule II :

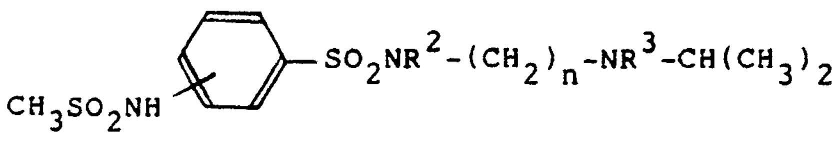 (II)

où $R^2$ est alcoyle en chaîne droite ou ramifiée comptant jusqu'à 6 atomes de carbone, mais non méthyle; $R^3$ est isopropyle; n est l'un des entiers 2, 3 et 4; ou un sel pharmaceutiquement acceptable de celui-ci.

5. Le N-(1-méthyléthyl)-N-[2-[bis(méthyléthyl)-amino]éthyl]-4-(méthylsulfonamidò)benzènesulfonamide ou un sel pharmaceutiquement acceptable de celui-ci.

6. Composé suivant la revendication 1 ou 2, dans lequel $R^3$ est hydrogène.

7. Composé de formule II :

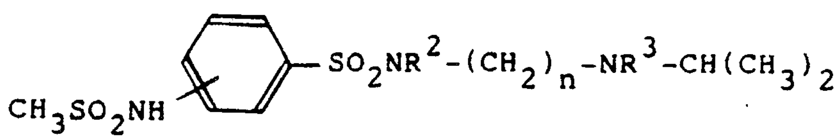 (II)

dans lequel $R^2$ est alcoyle en chaîne droite ou ramifiée comptant jusqu'à 6 atomes de carbone, mais non méthyle; $R^3$ est hydrogène; n est l'un des entiers 2, 3 et 4; ou un sel pharmaceutiquement acceptable de celui-ci.

8. Le N-(1-méthyléthyl)-N-(2-[(1-méthyléthyl)-amino]éthyl]-4-(méthylsulfonamido)benzènesulfonamide ou un sel pharmaceutiquement acceptable de celui-ci.

9. Procédé de préparation d'un composé de formule I :

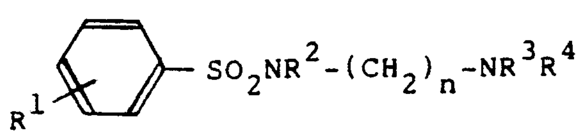 (I)

(où $R^1$, $R^2$, $R^3$ et $R^4$ sont tels que définis dans la revendication 1)
ou d'un sel pharmaceutiquement acceptable de celui-ci, dans lequel :
a) une amine de formule III :

$$H\overset{*}{N}R^2-(CH_2)_n-NR^4R^5 \qquad \text{(III)}$$

(où $R^2$, $R^4$ et n sont tels que définis ci-dessus et $R^5$ est $R^3$ ou un radical protecteur éliminable) est sulfonylée à l'atome d'azote marqué d'un astérisque par réaction avec un agent de sulfonylation pour introduire un radical sulfonyle de formule IV :

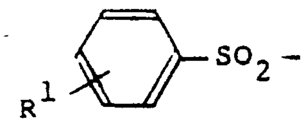

(IV)

(où $R^1$ est tel que défini ci-dessus)
et, lorsque la chose est nécessaire, un radical protecteur $R^5$ est éliminé;
ou
(b) une amine de formule V :

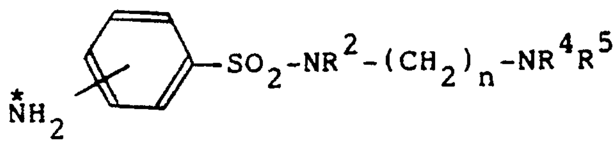

(V)

(où $R^2$, $R^4$, $R^5$ et n sont tels que définis ci-dessus)
est sulfonylée à l'atome d'azote marqué d'un astérisque par réaction avec un agent de sulfonylation pour introduire un radical alcoylsulfonyle comptant 1 à 6 atomes de carbone, un radical arylsulfonyle comptant 6 à 10 atomes de carbone ou un radical perfluoroalcoylsulfonyle comptant 1 à 6 atomes de carbone, et, lorsque la chose est nécessaire, un radical protecteur $R^5$ est éliminé;
ou
(c) une amine de formule VI :

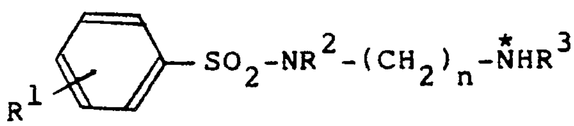

(VI)

(où $R^1$, $R^2$, n et $R^3$ sont tels que définis ci-dessus)
est alcoylée à l'atome d'azote marqué d'un astérisque par réaction avec un agent d'alcoylation pour introduire un radical $C_1$-$C_6$-alcoyle;
ou
(d) une amine de formule :

$$NHR^3R^4$$

(où $R^3$ et $R^4$ sont tels que définis ci-dessus)
est alcoylée à l'aide d'un agent d'alcoylation pour introduire un radical alcoyle substitué de formule VII :

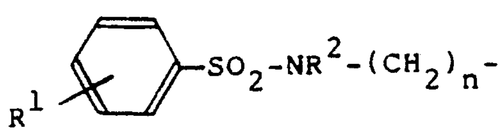

(VII)

(où $R^1$, $R^2$ et n sont tels que définis ci-dessus);
ou
(e) un composé carbonylé de formule :

$$C_mH_{2m+1}\text{-CO-}C_pH_{2p+1}$$

(où m et p sont indépendamment 0, 1, 2, 3, 4 ou 5 et le total de m et p est 0, 1, 2, 3, 4 ou 5) est soumis à une amination réductrice par réaction avec une amine de formule VI telle qu'indiquée et définie ci-dessus;

ou

(f) un aldéhyde de formule VIII :

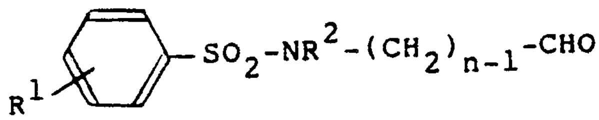

(VIII)

(où $R^1$, $R^2$ et n sont tels que définis ci-dessus)

est soumis à une amination réductrice par réaction avec une amine de formule :

$$NHR^3R^4$$

(où $R^3$ et $R^4$ sont tels que définis ci-dessus);

ou

(g) une base de Schiff de formule IX ou X :

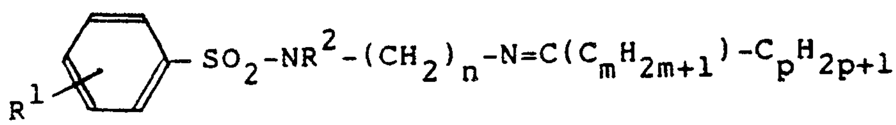

(IX)

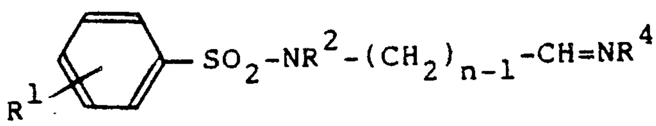

(X)

(où $R^1$, $R^2$, $R^4$, n, m et p sont tels que définis ci-dessus)

ou un amide de formule XI ou XII :

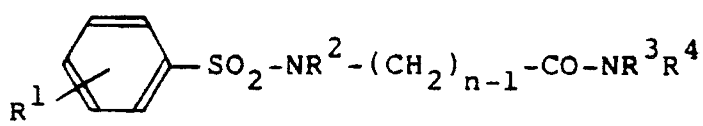

(XI)

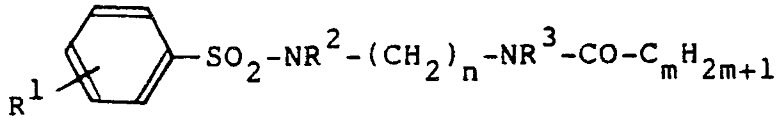

(XII)

(où $R^1$, $R^2$, $R^3$, $R^4$, n et m sont tels que définis ci-dessus) est réduit pour former une amine;

et, si la chose est souhaitée, un acide peut être ajouté à une forme base libre du composé de formule I pour donner un sel d'addition d'acide ou bien un sel d'addition d'acide peut être neutralisé par soustraction d'un acide pour donner une forme base libre du composé de formule I.

Revendications pour les Etats contractants suivants : AT, ES, GR

**1.** Procédé de préparation d'un composé de formule I :

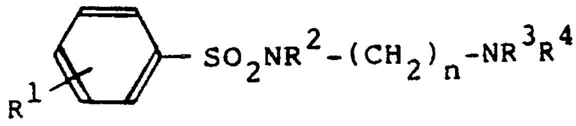

(I)

où $R^1$ est alcoylsulfonamido comptant 1 à 6 atomes de carbone; $R^2$ est alcoyle comptant jusqu'à 6 atomes de carbone, mais non méthyle; $R^3$ est hydrogène ou alcoyle comptant 1 à 6 atomes de carbone; $R^4$ est alcoyle de 1 à 6 atomes de carbone; et n est l'un des entiers 2, 3 et 4; ou d'un sel pharmaceutiquement acceptable de celui-ci; dans lequel :

a) une amine de formule III :

$$H\overset{*}{N}R^2-(CH_2)_n-NR^4R^5 \qquad (III)$$

(où $R^2$, $R^4$ et n sont tels que définis ci-dessus et $R^5$ est $R^3$ ou un radical protecteur éliminable) est sulfonylée à l'atome d'azote marqué d'un astérisque par réaction avec un agent de sulfonylation pour introduire un radical sulfonyle de formule IV :

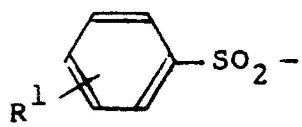

(IV)

(où $R^1$ est tel que défini ci-dessus)
et, lorsque la chose est nécessaire, un radical protecteur $R^5$ est éliminé;
ou
(b) une amine de formule V :

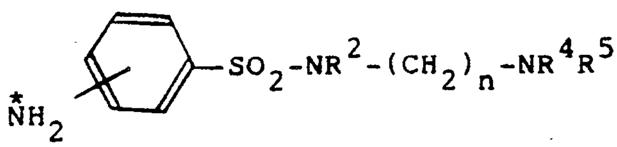

(V)

(où $R^2$, $R^4$, $R^5$ et n sont tels que définis ci-dessus)
est sulfonylée à l'atome d'azote marqué d'un astérisque par réaction avec un agent de sulfonylation pour introduire un radical alcoylsulfonyle comptant 1 à 6 atomes de carbone, un radical arylsulfonyle comptant 6 à 10 atomes de carbone ou un radical perfluoroalcoylsulfonyle comptant 1 à 6 atomes de carbone, et, lorsque la chose est nécessaire, un radical protecteur $R^5$ est éliminé;
ou
(c) une amine de formule VI :

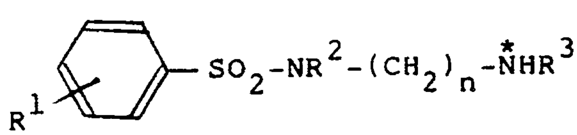

(VI)

(où $R^1$, $R^2$, n et $R^3$ sont tels que définis ci-dessus)
est alcoylée à l'atome d'azote marqué d'un astérisque par réaction avec un agent d'alcoylation pour introduire un radical $C_1$-$C_6$-alcoyle;
ou
(d) une amine de formule :

$$NHR^3R^4$$

(où $R^3$ et $R^4$ sont tels que définis ci-dessus)
est alcoylée à l'aide d'un agent d'alcoylation pour introduire un radical alcoyle substitué de formule VII :

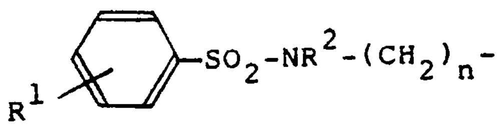
(VII)

(où $R^1$, $R^2$ et n sont tels que définis ci-dessus);
ou
(e) un composé carbonylé de formule :

$$C_mH_{2m+1}-CO-C_pH_{2p+1}$$

(où m et p sont indépendamment 0, 1, 2, 3, 4 ou 5 et le total de m et p est 0, 1, 2, 3, 4 ou 5)
est soumis à une amination réductrice par réaction avec une amine de formule VI telle qu'indiquée et définie ci-dessus;
ou
(f) un aldéhyde de formule VIII :

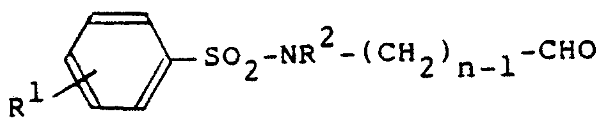
(VIII)

(où $R^1$, $R^2$ et n sont tels que définis ci-dessus)
est soumis à une amination réductrice par réaction avec une amine de formule :

$$NHR^3R^4$$

(où $R^3$ et $R^4$ sont tels que définis ci-dessus);
ou
(g) une base de Schiff de formule IX ou X :

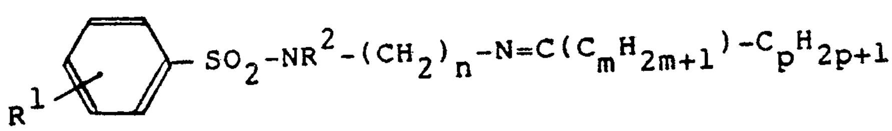
(IX)

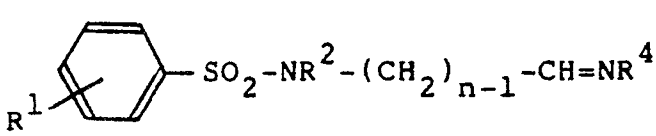
(X)

(où $R^1$, $R^2$, $R^4$, n, m et p sont tels que définis ci-dessus)
ou un amide de formule XI ou XII :

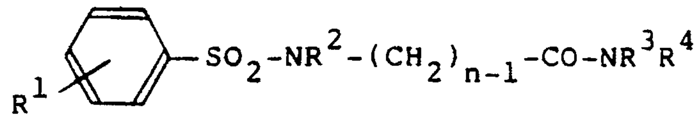

(XI)

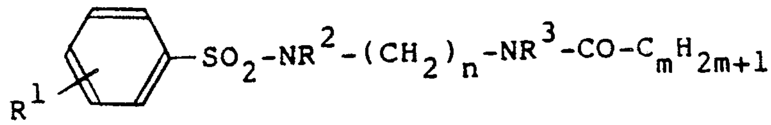

(XII)

(où $R^1$, $R^2$, $R^3$, $R^4$, n et m sont tels que définis ci-dessus)
est réduit pour former une amine;
et, si la chose est souhaitée, un acide peut être ajouté à une forme base libre du composé de formule I pour donner un sel d'addition d'acide ou bien un sel d'addition d'acide peut être neutralisé par soustraction d'un acide pour donner une forme base libre du composé de formule I.

2. Procédé suivant la revendication 1, dans lequel $R^3$ est hydrogène.

3. Procédé suivant la revendication 1 ou 2, dans lequel $R^3$ est alcoyle comptant 1 à 6 atomes de carbone.

4. Procédé suivant la revendication 2 ou 3, dans lequel $R^1$ est $CH_3SO_2NH$-.

5. Procédé de préparation d'une composition pharmaceutique, dans lequel un composé de formule I tel que défini et illustré dans la revendication 1, ou un sel pharmaceutiquement acceptable de celui-ci, est mis en combinaison ou en association avec un excipient pharmaceutiquement acceptable.

6. Procédé suivant la revendication 5, dans lequel $R^3$ est hydrogène.

7. Procédé suivant la revendication 5, dans lequel $R^3$ est alcoyle comptant 1 à 6 atomes de carbone.

8. Procédé suivant la revendication 6 ou 7, dans lequel $R^1$ est $CH_3SO_2NH$-.

## Ansprüche

1. Verbindung der Formel (I)

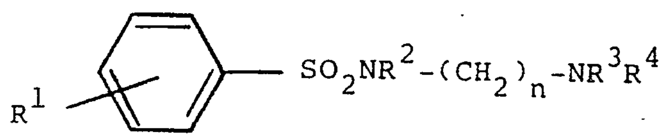

(I),

worin $R^1$ Alkylsulfonamido mit 1 bis 6 Kohlenstoffatomen ist; $R^2$ Alkyl mit bis zu 6 Kohlenstoffatomen, aber nicht Methyl bedeutet; $R^3$ Wasserstoff oder Alkyl mit 1 bis 6 Kohlenstoffatomen darstellt; $R^4$ Alkyl mit 1 bis 6 Kohlenstoffatomen bedeutet und n eine ganze Zahl von 2, 3 oder 4 ist, oder ein pharmazeutisch annehmbares Salzhievon.

2. Verbindung nach Anspruch 1, worin $R^1$ $CH_3SO_2NH$- ist.

3. Verbindung nach Anspruch 1 oder 2, worin $R^3$ Alkyl mit 1 bis 6 Kohlenstoffatomen ist.

4. Verbindung der Formel (II)

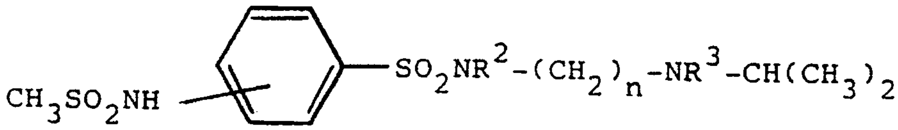

(II),

worin $R^2$ gerad- oder verzweigtkettiges Alkyl mit bis zu 6 Kohlenstoffatomen, aber nicht Methyl bedeutet; $R^3$ Isopropyl darstellt und n eine ganze Zahl von 2, 3 oder 4 ist, oder ein pharmazeutisch annehmbares Salz hievon.

5. N-(1-Methyläthyl)-N-[2-[bis-(methyläthyl)-amino]-äthyl]-4-(methylsulfonamido)-benzolsulfonamid oder ein pharmazeutisch annehmbares Salz hievon.

6. Verbindung nach Anspruch 1 oder 2, worin $R^3$ Wasserstoff ist.

7. Verbindung der Formel (II)

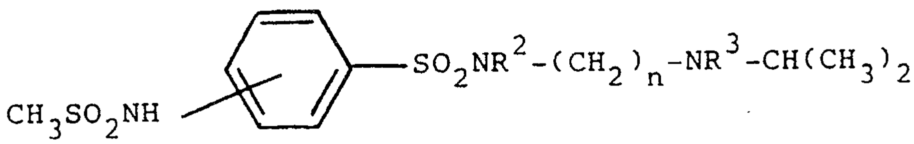

(II),

worin $R^2$ gerad- oder verzweigtkettiges Alkyl mit bis zu 6 Kohlenstoffatomen, aber nicht Methyl bedeutet; $R^3$ Wasserstoff darstellt und n eine ganze Zahl von 2, 3 oder 4 ist, oder ein pharmazeutisch annehmbares Salz hievon.

8. N-(1-Methyläthyl)-N-[2-[bis-(methyläthyl)-amino]-äthyl]-4-(methylsulfonamido)-benzolsulfonamid oder ein pharmazeutisch annehmbares Salz hievon.

9. Verfahren zur Herstellung einer Verbindung der Formel (I)

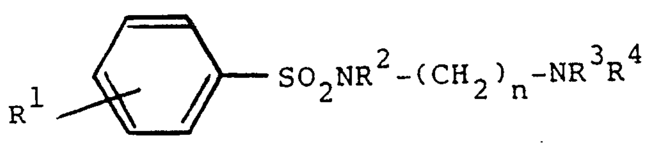

(I)

(worin $R^1$, $R^2$, $R^3$, $R^4$ und n wie in Anspruch 1 definiert sind) oder eines pharmazeutisch annehmbaren Salzes hievon, worin

a) ein Amin der Formel (III)

$$H\overset{*}{N}R^2-(CH_2)_n-NR^4R^5 \qquad (III)$$

(worin $R^2$, $R^4$ und n wie oben definiert sind und $R^5$ die Bedeutung $R^3$ hat oder eine entfernbare Schutzgruppe ist) an dem mit einem Sternchen bezeichneten Stickstoffatom durch Umsetzen mit einem Sulfonylierungsmittel zum Einführen einer Sulfonylgruppe der Formel (IV)

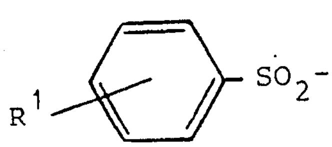

(IV)

(worin $R^1$ wie oben definiert ist) sulfonyliert und, wenn notwendig, eine Schutzgruppe $R^5$ entfernt wird oder

(b) ein Amin der Formel (V)

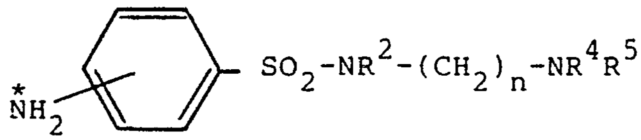

(V)

(worin $R^2$, $R^4$, $R^5$ und n wie oben definiert sind) an dem mit einem Sternchen bezeichneten Stickstoffatom durch Umsetzen mit einem Sulfonylierungsmittel zum Einführen einer Alkylsulfonylgruppe mit 1 bis 6 Kohlenstoffatomen, einer Arylsulfonylgruppe mit 6 bis 10 Kohlenstoffatomen oder einer Perfluoralkylsulfonylgruppe mit 1 bis 6 Kohlenstoffatomen sulfonyliert und, wenn notwendig, eine Schutzgruppe $R^5$ entfernt wird oder

(c) ein Amin der Formel (VI)

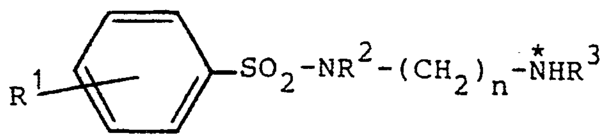

(VI)

(worin $R^1$, $R^2$, n und $R^3$ wie oben definiert sind) an dem mit einem Sternchen bezeichneten Stickstoffatom durch Umsetzen mit einem Alkylierungsmittel zum Einführen einer $C_1$-$C_6$-Alkylgruppe alkyliert wird oder

(d) ein Amin der Formel $NHR^3R^4$ (worin $R^3$ und $R^4$ wie oben definiert sind) mit einem Alkylierungsmittel zum Einführen einer substituierten Alkylgruppe der Formel (VII)

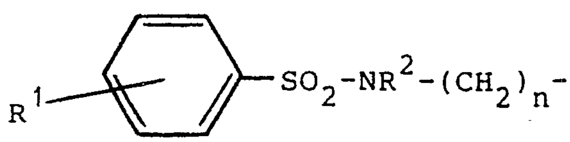

(VII)

(worin $R^1$, $R^2$ und n wie oben definiert sind) alkyliert wird oder

(e) eine Carbonylverbindung der Formel $C_mH_{2m+1}$-$CO_pH_{2p+1}$(worin m und p unabhängig Null, 1, 2, 3, 4 oder 5 sind und die Gesamtsumme von m und p Null, 1, 2, 3, 4 oder 5 beträgt) durch Umsetzen mit einem Amin der Formel (VI), wie oben gezeigt und definiert, reduktiver Aminierung unterworfen wird oder

(f) ein Aldehyd der Formel (VIII)

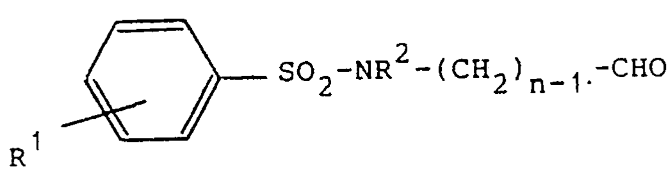

(VIII)

( worin $R^1$, $R^2$ und n wie oben definiert sind) durch Umsetzen mit einem Amin der Formel $HNR^3R^4$ (worin $R^3$ und $R^4$ wie oben definiert sind) reduktiver Aminierung unterworfen wird oder

(g) eine Schiff'sche Base der Formel (IX) oder (X)

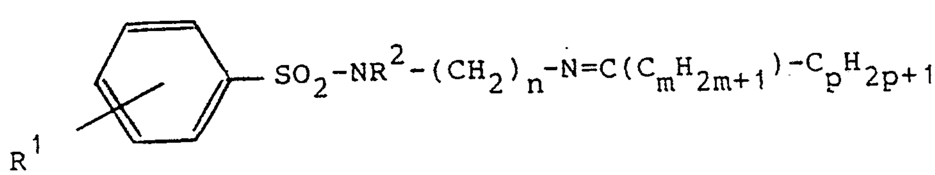

(IX)

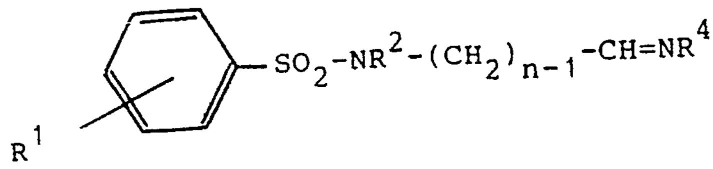

(X)

(worin $R^1$, $R^2$, $R^4$, n, m und p wie oben definiert sind) oder ein Amid der Formel (XI) oder (XII)

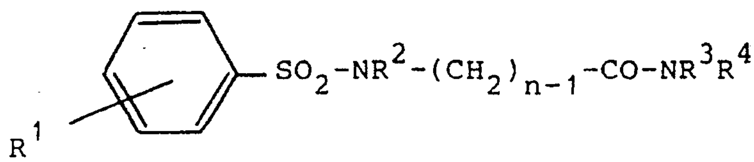

(XI)

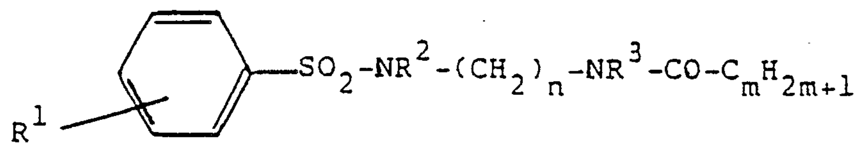

(XIII)

(worin $R^1$, $R^2$, $R^3$, $R^4$, n und m wie oben definiert sind) unter Bildung eines Amins reduziert wird und,

wenn gewünscht, einer freien Basenform der Verbindung der Formel (I) eine Säure unter Bildung eines Säureadditionssalzes zugesetzt oder ein Säureadditionssalz durch Subtraktion einer Säure unter Bildung einer freien Basenform der Verbindung der Formel (I) neutralisiert werden kann.

Patentansprüche für folgende Vertragsstaaten : AT, ES, GR

**1.** Verfahren zur Herstellung einer Verbindung der Formel (I)

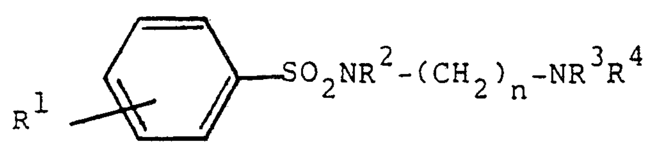

(I)

oder eines pharmazeutisch annehmbaren Salzes hievon, worin $R^1$ Alkylsulfonamido mit 1 bis 6 Kohlenstoffatomen ist; $R^2$ Alkyl mit bis zu 6 Kohlenstoffatomen, aber nicht Methyl bedeutet; $R^3$ Wasserstoff oder Alkyl mit 1 bis 6 Kohlenstoffatomen darstellt, $R^4$ Alkyl mit 1 bis 6 Kohlenstoffatomen bedeutet und n eine ganze Zahl von 2, 3 oder 4 ist, worin

a) ein Amin der Formel (III)

$$H\overset{*}{N}R^2-(CH_2)_n-NR^4R^5 \qquad (III)$$

(worin $R^2$, $R^4$ und n wie oben definiert sind und $R^5$ die Bedeutung $R^3$ hat oder eine entfernbare Schutzgruppe ist) an dem mit einem Sternchen bezeichneten Stickstoffatom mit einem Sulfonylierungsmittel zum Einführen einer Sulfonylgruppe der Formel (IV)

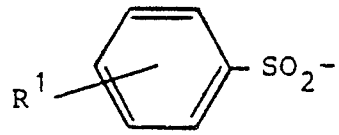

(IV)

(worin $R^1$ wie oben definiert ist) sulfonyliert und, wenn notwendig, eine Schutzgruppe $R^5$ entfernt wird oder

(b) ein Amin der Formel (V)

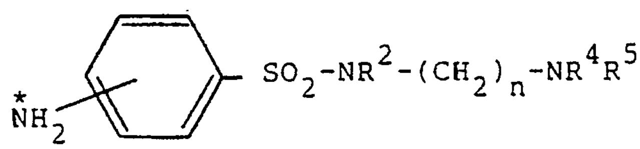

(V)

(worin $R^2$, $R^4$, $R^5$ und n wie oben definiert sind) an dem mit einem Sternchen bezeichneten Stickstoffatom durch Umsetzen mit einem Sulfonylierungsmittel zum Einführen einer Alkylsulfonylgruppe mit 1 bis 6 Kohlenstoffatomen, einer Arylsulfonylgruppe mit 6 bis 10 Kohlenstoffatomen oder einer Perfluoralkylsulfonylgruppe mit 1 bis 6 Kohlenstoffatomen sulfonyliert und, wenn notwendig, eine Schutzgruppe $R^5$ entfernt wird oder

(c) ein Amin der Formel (VI)

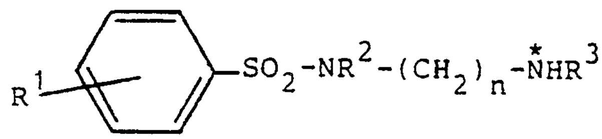

(VI)

(worin $R^1$, $R^2$, n und $R^3$ wie oben definiert sind) an dem mit einem Sternchen bezeichneten Stickstoffatom durch Umsetzen mit einem Alkylierungsmittel zum Einführen einer $C_1$-$C_6$-Alkylgruppe alkyliert wird oder

(d) ein Amin der Formel $NHR^3R^4$ (worin $R^3$ und $R^4$ wie oben definiert sind) mit einem Alkylierungsmittel zum Einführen einer substituierten Alkylgruppe der Formel (VII)

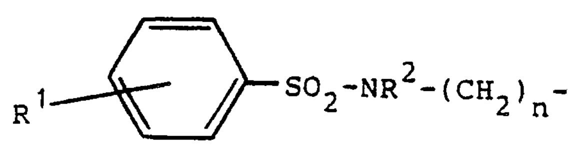

(VII)

(worin $R^1$, $R^2$ und n wie oben definiert sind) alkyliert wird oder

(e) eine Carbonylverbindung der Formel $C_mH_{2m+1}$-CO-$C_pH_{2p+1}$(worin m und p unabhängig Null, 1, 2, 3, 4 oder 5 sind und die Gesamtsumme von m und p Null, 1, 2, 3, 4 oder 5 beträgt) durch Umsetzen mit einem Amin der Formel (VI), wie oben gezeigt und definiert, reduktiver Aminierung unterworfen wird oder

(f) ein Aldehyd der Formel (VIII)

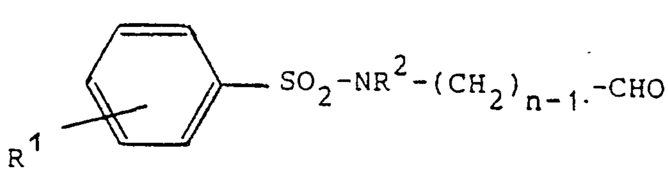

(VIII)

(worin $R^1$, $R^2$ und n wie oben definiert sind) durch Umsetzen mit einem Amin der Formel $HNR^3R^4$ (worin $R^3$ und $R^4$ wie oben definiert sind) reduktiver Aminierung unterworfen wird oder

(g) eine Schiff'sche Base der Formel (IX) oder (X)

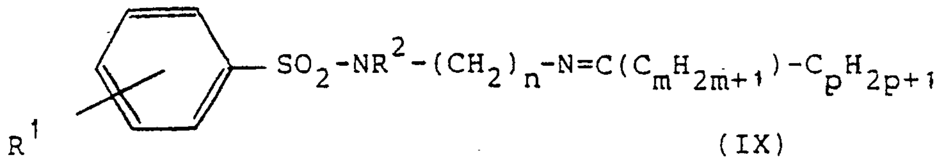 (IX)

(IX)

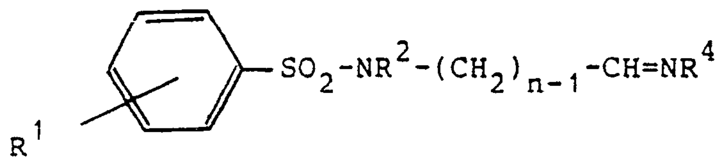 (X)

(worin $R^1$, $R^2$, $R^4$, n, m und p wie oben definiert sind) oder ein Amid der Formel (XI) oder (XII)

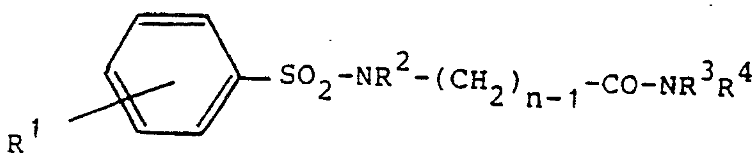 (XI)

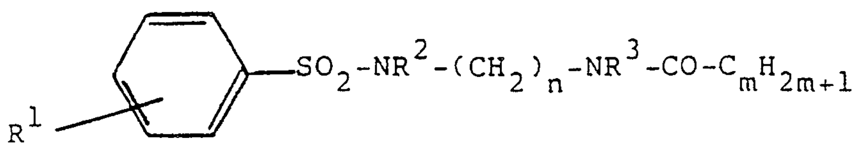 (XIII)

(worin $R^1$, $R^2$, $R^3$, $R^4$, n und m wie oben definiert sind) unter Bildung eines Amins reduziert wird und, wenn gewünscht, einer freien Basenform der Verbindung der Formel (I) eine Säure unter Bildung eines Säureadditionssalzes zugesetzt oder ein Säureadditionssalz durch Subtraktion einer Säure unter Bildung einer freien Basenform der Verbindung der Formel (I) neutralisiert werden kann.

2. Verfahren nach Anspruch 1, worin $R^3$ Wasserstoff ist.

3. Verfahren nach Anspruch 1 oder 2, worin $R^3$ Alkyl mit 1 bis 6 Kohlenstoffatomen ist.

4. Verfahren nach Anspruch 2 oder 3, worin $R^1$ $CH_3SO_2NH$- ist.

5. Verfahren zur Herstellung einer pharmazeutischen Zusammensetzung, worin eine Verbindung der Formel (I), wie in Anspruch 1 definiert und illustriert, oder ein pharmazeutisch annehmbares Salz hievon mit einem pharmazeutisch annehmbaren Träger kombiniert oder vereinigt wird.

6. Verfahren nach Anspruch 5, worin $R^3$ Wasserstoff ist.

7. Verfahren nach Anspruch 5, worin $R^3$ Alkyl mit 1 bis 6 Kohlenstoffatomen ist.

8. Verfahren nach Anspruch 6 oder 7, worin $R^1$ $CH_3SO_2NH$- ist.